Europäisches Patentamt

European Patent Office

Office européen des brevets

(19)

(11) Publication number: 0 132 971
A2

# (12) EUROPEAN PATENT APPLICATION

(21) Application number: 84304579.0

(22) Date of filing: 04.07.84

(51) Int. Cl.⁴: C 07 C 17/156
F 23 G 7/00, F 23 C 9/08
F 23 M 5/08

(30) Priority: 27.07.83 GB 8320220

(43) Date of publication of application:
13.02.85 Bulletin 85/7

(84) Designated Contracting States:
AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Newman, Michael Wesley
5 Cherry Tree Avenue
Barnton Northwich Cheshire(GB)

(72) Inventor: Sealey, Charles
Tilburgseweg 228
Breda (N. Br.)(NL)

(72) Inventor: Simmonds, Frank
9 Leabank Drive
Worcester Worcs.(GB)

(74) Representative: Oldroyd, Alan et al,
Imperial Chemical Industries PLC Legal Department:
Patents PO Box 6 Bessemer Road
Welwyn Garden City Herts, AL7 1HD(GB)

(54) Production of chlorinated hydrocarbons.

(57) An oxychlorination process for chlorinated hydrocarbons in which at least part of the feedstock hydrogen chloride is provided by combustion of chlorine-containing organic residues in an oxygen-rich flame, the combusted gases being transferred from the combustion chamber to the oxychlorination reaction above the dewpoint of their water content, thus avoiding the need for condensing and reheating. Also a combustion chamber therefor with novel cooler design to give the required flame and temperature control, and oxychlorination apparatus comprising this chamber.

## PRODUCTION OF CHLORINATED HYDROCARBONS

This invention relates to the production of chlorinated hydrocarbons by an oxychlorination reaction using waste chlorine-containing residues to provide the hydrogen chloride therefor. The invention also relates to apparatus for producing the chlorinated hydrocarbons.

Chlorine-containing organic compounds are formed as waste residues in various manufacturing processes, and need to be destroyed in an environmentally acceptable manner. It has been proposed in US Patent 4 233 280 to burn the residues in oxygen to produce hydrogen chloride, carbon dioxide, carbon monoxide and water.

According to further proposals in US Patent 4 233 280 a water-jacketed combustion chamber may be used to cool the combustion gases to between 200-800°C before they are quenched in an aqueous hydrogen chloride solution, the latter also serving as a washing medium to remove iron which is usually present as an undesirable impurity; the emergent wet hydrogen chloride is corrosive and cooled to condense out the water and provide an anhydrous hydrogen chloride feedstock for use in further industrial processes, such as an oxychlorination process. In an oxychlorination process hydrogen chloride gas is reacted with an unsaturated organic compound and an oxygen-containing gas, in the presence of a catalyst, to produce useful chlorinated hydrocarbons. In this way, for example, chlorine-containing waste residues from the manufacture of vinyl chloride by the cracking of ethylene dichloride may be combusted in oxygen or an oxygen-rich atmosphere to recover the chlorine as hydrogen chloride, which, after quenching, washing and drying, may be reacted with ethylene in an oxychlorination process to produce more ethylene dichloride.

The oxychlorination reaction is an exothermic reaction carried out at elevated temperatures and pressures, typically at 240°C and 6-7 bar pressure in the chlorination of ethylene, for example. In practice we have discovered that it is not necessary to dry the combustion gases before they are used as a souce of hydrogen chloride in such oxychlorination reactions, provided there is no condensation of the water. On the basis of this discovery we have devised a new cycle for combining residue combustion with an oxychlorination reaction, which avoids inefficient procedures hitherto considered necessary.

According to a first aspect of the present invention, there is provided a process for producing chlorinated hydrocarbon compounds by reacting an unsaturated organic compound with hydrogen chloride and oxygen in an oxychlorination reaction for which at least part of the hydrogen chloride is provided by the combustion in a combustion chamber of chlorine-containing organic residues in an oxygen-rich flame to produce gases which include hydrogen chloride and water vapour, characterised in that the gases are transferred from the combustion chamber to the oxychlorination reaction at a temperature above the dew point of the water vapour therein.

In order to enable the present process to be carried out, there is also provided according to a second aspect of the invention, an apparatus for producing chlorinated hydrocarbon compounds, comprising a combustion chamber for combusting chlorine-containing organic residues in an oxygen-rich flame to produce combustion gases which include hydrogen chloride and water vapour and which are fed to an oxychlorination plant for reacting an unsaturated organic compound with oxygen and hydrogen chloride, characterised in that the combustion chamber has means for controlling the temperature of the effluent combusted gases such that they are transferred to the oxychlorination plant at a temperature above the dew point of the water vapour therein.

In general, to help to ensure that the effluent gases are maintained at a temperature above the dew point of the water therein throughout their transfer to the oxychlorination plant the combustion chamber has an outlet for the combusted gases which is connected directly to an inlet port in the oxychlorination plant by lagged and preferably heat-traced ducting.

In a preferred embodiment of this aspect of the present invention, the combustion of the chlorine-containing residues is effected at high flame temperatures. The means for controlling the temperature of the effluent combusted gases should therefore then also control that temperature below the limiting temperature of the outlet ducting material when in contact with the effluent gases. The chamber should also have means for preventing the direct inpringement of the combustion flame on any internal structure of the chamber, and for preventing the direct inpingement of heat radiation from the flame onto any part of the walls of the chamber. These latter means for preventing the inpingement of the flame or its radiation may also at least partially control the temperature of the effluent gases.

As in previously known processes, it is desirable in the present process to remove the metal impurities (mainly iron) which are usually present in chlorine-containing residues, before the combustion gases are fed to the oxychlorination plant. A preferred process according to the invention is one wherein the residues are treated with an aqueous solution of mineral acid before they are fed to the combustion chamber. This may be carried out in plurality of stages, as required, but by being carried out before combustion, the temperature and pressure of the combusted gases can each be maintained at a level which is directly compatible with the oxychlorination process as described above.

In a third aspect the present invention provides a combustion chamber for combusting chlorine-containing organic residues to produce combustion gases which include hydrogen chloride and water vapour, said chamber having an exit port, characterised in that the combustion chamber has means for controlling the temperature of the effluent combusted gases at the exit port above the dew point of the water vapour therein.

In the chamber of the present invention the residues are combusted in an oxygen-rich atmosphere at elevated pressures. As the combusted gases do not go through the washing stage of the prior art, it is desirable to avoid excessive formation of carbon monoxide and soot. The proportion of oxygen to the organic residues and any other combustible material is preferably maintained at least sufficiently high to provide for complete combustion of all carbonaceous material to carbon dioxide.

The flame temperature, for example as measured by radiation pyrometer for example, is preferably in excess of 2000°C to ensure complete destruction of the residues. A preferred process includes the addition of a hydrogen-containing support fuel to the combustion mixture at a rate sufficient to raise the flame temperature above 2000°C.

The combustion of chlorine-containing organic residues is usually carried out at pressures of 1-20 bar. The oxychlorination reaction is also usually carried out at elevated pressures, typically 6-7 bar. The present invention does not require the loss of the elevated combustion pressure and subsequent recompression, as did previous processes, and we prefer that the combustion of the organic residues be carried out at a pressure greater than oxychlorination, thereby to enable the combusted gases to be fed directly to the oxychlorination reaction without any intermediate compression stage. However, even where the oxychlorination pressure is greater than the combustion pressure, the compression stage required will be less than that required when the combustion pressure is released.

Conventional refractory and metallic materials may be used in the construction of the combustion chamber of the present invention. In such a case, although the flame temperature within the combustion chamber is preferably in excess of 2000°C, it is important, in order to avoid undue corrosion of such materials, for the gases elsewhere within the chamber to be at a much lower temperature, particularly where they contact the materials used in the construction of the chamber. In the presence of hydrogen chloride the limiting temperatures of conventional materials, such as refractory materials and metallic alloys are substantially lower than those tolerable in its absence, for example, in the presence of hydrocarbon combustion gas products. Steps must therefore be taken where conventional materials are used to avoid direct impingement of the hot flame on any internal structure of the chamber and to maintain suface temperatures within recognised limits for these materials in the presence of hydrogen chloride.

The effluent gases may be led to an oxychlorination reactor through an exit port in the combustion chamber which may be of a conventional refractory material, and in ducting of a conventional metallic material. Thus, whilst the temperature of the effluent combusted gases should be above the dew point of the water vapour therein it should be below the limiting temperature of the chamber material defining the exit port when in contact with the effluent gases, or of the effluent ducting material when in contact with the effluent gases. Both the dew point and the limiting temperature of any give port or ducting materials in the operational conditions of the reactor will be known to the skilled man.

In a preferred embodiment of the combustion chamber of the invention, therefore, the means for controlling the temperature of the effluent combusted gases should therefore also be capable of controlling that temperature below the limiting temperature of the chamber material defining the exit port.

In another preferred embodiment the chamber should therefore also have means for preventing the direct impingement of the combustion flame on any internal structure of the chamber, and for preventing the direct impingement of heat radiation from the flame on any part of the refractory walls of the chamber.

To achieve the desired temperature ranges for the internal structures of the combustion chamber we prefer to use a refractory walled combustion chamber equipped with baffling and controllable cooling means for removing heat by circulation of fluid therethrough, water being a suitable coolant, the baffling and cooling means being part of the chamber and located within the chamber, and being disposed so as to prevent the direct impingement of heat radiation from the flame on to any part of the refractory walls of the chamber and to baffle the combusted gases to prevent direct impingment of the flame on any internal structure of the chamber. This is to ensure that the temperatures experienced by the refractories and the baffling and cooling means themselves are within acceptable limits.

We prefer to use a baffling and cooling means comprising at least one lining mounted within the chamber such that it shields at least part of the refractory walls of the chamber from direct impingement of heat radiation from the flame and prevents direct impingement of the flame onto that part of the refractory walls and itself, and is hollow for ducting coolant. A particular baffling and cooling means is one in which the lining of this type is formed from a coiled tube for ducting coolant, to increase the heat transfer area between the combusted gases and the coolant.

The temperature of the combusted gases as they leave the combustion chamber is preferably in the range 350-550°C. The gases may then be conveyed for example in stainless steel piping to the oxychlorination plant. The elevated temperature enables the gases to be fed directly into the oxychlorination reactor without any intermediate preheating stage such as had been necessary with the previous quench and chill-dry methods. This enables substantial savings in energy to be made, leading to a more efficient cycle.

However, if the above preferred gas temperatures are to be reached before the gases are transferred to the oxychlorination plant, further cooling beyond that needed simply to protect the refractory walls is required. It is also desirable to maximise recovery of the energy from the combustion, but high pressure operations using oxygen rather than air lead to a lower volumetric flow of combustion gases, which tends to hamper the required cooling. In view of these considerations we prefer to use a further controllable cooling means comprising a plurality of linings, the linings being spaced apart and mounted within the chamber such that the combusted gases are directed to flow between adjacent linings, and being hollow for coolant fluid circulation therethrough. In particular the linings may be mounted such that the combusted gases are directed to flow in one direction between adjacent linings and subsequently in an opposite direction. In particular each lining may surround and/or be surrounded by an adjacent lining.

A particularly preferred further cooling means is one in which the linings of this type are each formed from a helically coiled tube, thereby to cause the coolant fluid to flow along a helical path around the chamber, and also to increase the heat transfer area between the combusted gases and the coolant fluid.

Integers of the baffling and cooling means may also be integers of the further cooling means. For example, a lining in a further cooling means, which comprises linings where each lining surrounds and/or is surrounded by an adjacent lining, may also shield the refractory walls of the chamber from flame heat and baffle the combusted gases to prevent impingement of the flame on the walls or lining. Similarly a lining in a baffling and cooling means may also provide further cooling of the combusted gas.

A preferred combustion chamber according to the present invention is elongate and has

a)     a first end with an inlet port and a burner mounted thereat so as to direct the flame of the combusting gases along the axis of the chamber towards a closed second end of the chamber, the first and second ends of the chamber each having respectively a first end lining and a second end lining, which linings are hollow to carry coolant fluid,

b)     a side wall with at least one pair of linings consisting of an outer lining which surrounds an inner lining, which linings are spaced apart from each other and from the side wall to allow for gas flow therebetween and are hollow to carry coolant fluid, the outer lining being sealed to the first end lining and the inner lining being sealed to the second end lining, and

c)     an exit port through the side wall intermediate the two ends,

whereby the gases emerging from the burner flame towards the second end are caused to return towards the first end over the inner surface of the inner lining, and on reaching the first end lining are caused to flow between the inner and outer linings towards the second end, to reverse at the second end lining and flow over the outer surface of the outer lining before emerging through the exit port.

The hot combustion gases are thus cause to flow over at least four surfaces in heat exchange relationship with coolant fluid flowing through the inner and outer linings. Further pairs of similarly disposed inner and outer linings can be added to provide additional surface area, although we do not generally feel this to be necessary. Usually the chamber and the pair(s) of linings will be generally axially symmetric.

The first reversal of the gas flow, i.e. back towards the first (burner) end, serves to contain the radiating flame and prevent its impingement on the linings. If these are constructed of metallic walls with circulating cooling fluid, the outside metal skin temperatures will be governed principally by the coolant temperature even though they experience direct radiant heat input from the flame. Therefore conventional high nickel alloys (eg Incoloy and Inconel) may be used for these components.

The refractory walls are fully shielded from direct radiation and the temperatures thus ensuing enable conventional alumina based materials to be used.

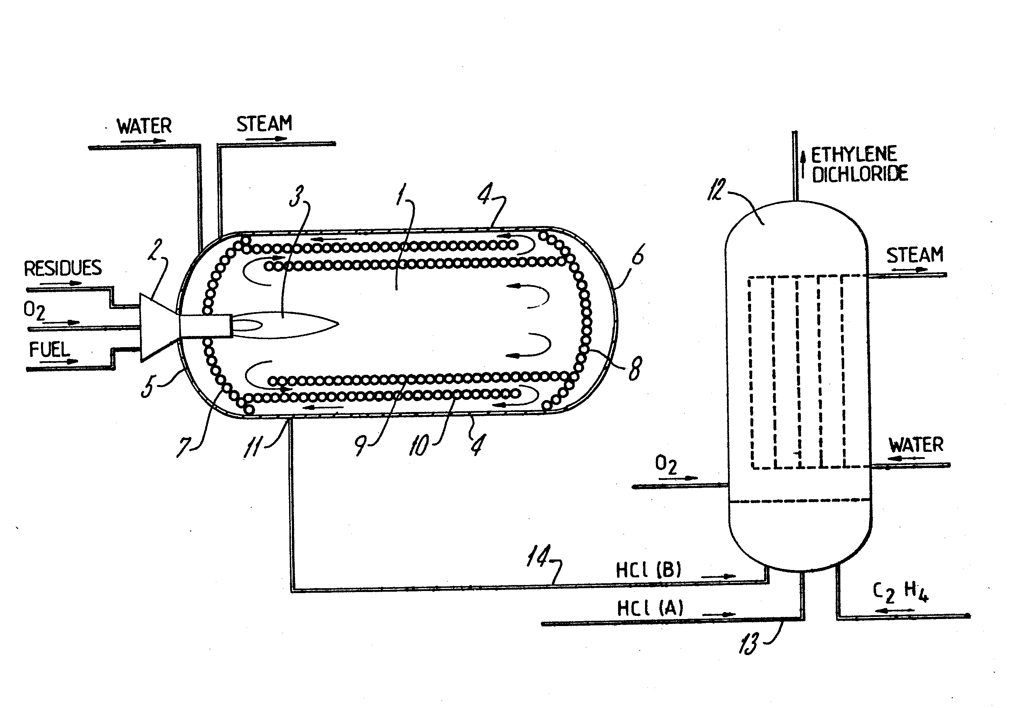

The invention is illustrated by an embodiment shown as a schematic sketch in the accompanying drawing. In this embodiment a generally cylindrical combustion chamber 1 has a burner 2 mounted at its first end 5 so as to direct a flame 3 of combusting gases down the axis of the chamber, towards its second end 6. The combusting gases comprise chlorine-containing residues, oxygen and, preferably a support fuel. The side wall 4 and ends 5 and 6 are formed of refractory materials. Mounted to cover the first (burner) end 5 of the chamber and also the second end 6 are first and second end protective linings respectively 7, 8 of coiled metal tubes. Along the sides are spaced an inner lining 9 surrounded by an outer lining 10 each formed of helically coiled tubes. The outer lining 10 is sealed to the wall 4 by being sealed to the first end protective lining 7 near the first (burner) end 5, but is not sealed to the wall 4 near the second end 6 to allow passage of combusted gases between itself and the wall 4. The inner lining 9, however, is spaced from and not sealed to the first protective end lining 7 near the first (burner) end 5, but is sealed to the second end protective lining 8 near the second end 6. An outlet port 11 is provided through the refractory side wall 4, between the two ends 5 and 6 but adjacent to the first (burner) end 5.

Also shown in the drawing is an oxychlorination plant 12 for converting ethylene to ethylene dichloride, using oxygen and hydrogen chloride. This plant is a standard plant with ethylene, hydrogen chloride and oxygen supplied at elevated temperatures. However, in addition to the normal hydrogen chloride feed (Hydrogen Chloride (A)) via the lower pipe 13, a further hydrogen chloride feed (Hydrogen Chloride (B)) is provided by the upper pipe 14 from the outlet port 11 of the combustion chamber. All the feed pipes are lagged and upper pipe 14 is heat-traced, the heat tracing being provided by a steam coil.

In use of the combustion chamber 1 a coolant liquid, eg water, is passed through the tubes forming the linings 7-10. The chlorine-containing residues, oxygen, and optionally a fuel gas (depending on the residues used) are fed to the burner 2 and combusted. The combusted gases are blown along the axis of the chamber 1 towards the closed second end 6. The end lining 8 sealed to the inner lining 9 forces the gases to return towards the first (burner) end 5 by travelling over the inner surface of the inner lining 9 as shown by the inner arrows. Thus they contain the flame and hold it clear of the inner lining surface. As further gases are forced in and burned, the combusted gases, on reaching the first end lining 7, are caused to reverse their direction of flow once more and to travel between the inner and outer linings 9 and 10, then back again, between the outer lining 10 and the refractory side wall 4 until they reach the exit port 11. In so travelling, the hot gases flow over four heat exchange surfaces, and are thereby firmly controlled in temperature by the time they reach the outlet port 11.

Within the chamber, the chlorinated residues combust in oxygen to form mainly hydrogen chloride, water vapour, carbon dioxide and/or carbon monoxide, when the flame temperature is above 2000°C and particularly when it is above 2500°C. To achieve such high temperatures, hydrogen-containing or other fuels may be added when necessary. The combusted gases are suitably cooled by the linings to about 400°C before they reach the outlet port 11, converting some of the coolant water to steam. The upper feed pipe 14 from the outlet port of the combustion chamber to the oxychlorination reactor is suitably lagged to maintain the combusted gases at elevated temperatures, thereby to maintain the water vapour therein above its dew point, even under the elevated pressures achieved in the combustion chamber. By keeping all water as vapour, corrosion problems are minimised, and by avoiding cooling of the combusted gases, eg for washing and water removal in known manner, further energy does not have to be found to reheat them prior to their being fed to the oxychlorination plant.

CLAIMS

1.     Process for producing chlorinated hydrocarbon
compounds by reacting an unsaturated organic compound
with hydrogen chloride and oxygen in an oxychlorination
reaction for at least part of which the hydrogen chloride
is provided by the combustion in a combustion chamber of
chlorine-containing organic residues in an oxygen-rich
flame to produce gases which include hydrogen chloride
and water vapour, characterised in that the gases are
transferred to the oxychlorination reaction, at a
temperature above the dew point of the water vapour
therein.

2.     Process according to claim 1, characterised in
that any metallic impurities present in the chlorine-
containing residues are removed by treatment of the
residues with an aqueous solution of mineral acid before
feeding to the combustion chamber.

3.     Process according to claim 1 or 2, characterised
in that the flame temperature used in excess of 2000°C.

4.     Process according to any one of the preceding
claims, characterised in that a hydrogen-containing
support fuel is added to the combustion mixture.

5.     Process according to any of the preceding claims,
characterised in that the combustion of the organic
residues is carried out at a pressure of 1 to 20 bar.

6.     Process according to any one of the preceding
claims, characterised in that combustion of the organic
residues is carried at a pressure which is greater than
that of the oxychlorination reaction into which the
combustion products are fed.

7.     Process according to any one of the preceding
claims, characterised in that the temperature of the combusted gases as they leave the combustion chamber is in the range 350-550°C.

8.      Combustion chamber suitable for use in a process according to any one of claims 1 to 7, said chamber having an exit port, characterised in that the combustion chamber has means for controlling the temperature of the effluent combusted gases at the exit port above the dew point of the water vapour therein.

9.      Combustion chamber according to claim 8 characterised in that the means for controlling the temperature of the effluent combusted gases is also capable of controlling that temperature below the limiting temperature of the chamber material defining the exit port when in contact with the effluent gases.

10.     Combustion chamber according to claim 8 or 9, characterised in that the means for controlling the temperature of the effluent combusted gases comprises a plurality of linings, the linings being spaced apart and mounted within the chamber such that the combusted gases are directed to flow between adjacent linings, and being hollow for circulation of coolant fluid therethrough.

11.     Combustion chamber according to claim 10, characterised in that the combusted gases are directed to flow in one direction between adjacent linings and subsequently in an opposite direction.

12.     Combustion chamber according to claim 10 or 11, characterised in that each lining surrounds and/or is surrounded by an adjacent lining.

13.     Combustion chamber according to any one of claims 10 to 12 characterised in that the linings are each formed from a helically coiled tube.

14. Combustion chamber according to any one of claims 8 to 13, characterised in that the chamber is refractory-walled and also has means for preventing the direct impingement of heat radiation from the flame onto any part of the refractory walls of the chamber and preventing direct impingement of the flame on any internal structure of the chamber.

15. Combustion chamber according to claim 14, characterised in that the means comprises at least one lining mounted within the chamber such that it shields at least part of the refractory walls of the chamber from direct impingment of heat refraction from the flame and prevents direct impingment of the flame on that part of the refractory walls and itself, and is hollow for ducting coolant.

16. Combustion chamber according to claim 15, characterised in that the lining is formed from a coiled tube.

17. Combustion chamber according to any one of claims 14 to 16 characterised in that the means comprises a plurality of linings, each lining surrounding and/or being surrounded by an adjacent lining.

18. Combustion chamber according to claim 17, characterised in that each such lining is formed from a helically coiled tube.

19. Combustion chamber according to any one of claims 8 to 18, characterised in that it is elongate and has

a)      a first end with an inlet port and a burner mounted thereat so as to direct the flame of the combusting gases along the axis of the chamber towards a closed second end of the chamber, the first and second ends of the chamber each having respectively a first end lining and a second end lining, which linings are hollow to carry coolant fluid,

b)      a side wall with at least one pair of linings consisting of an outer lining which surrounds an inner lining, which linings are spaced apart from each other and from the side wall to allow for gas flow therebetween and are hollow to carry coolant fluid, the outer lining being sealed to the first end lining and the inner lining being sealed to the second end lining, and

c)      an exit port through the side wall intermediate the two ends,

whereby the gases emerging from the burner flame towards the second end are caused to return towards the first end over the inner surface of the inner lining, and on reaching the first end lining are caused to flow between the inner and outer linings towards the second end, to reverse at the second end lining and flow over the outer surface of the outer lining before emerging through the exit port.

20.     Apparatus for producing chlorinated hydrocarbon compounds, comprising a combustion chamber for combusting chlorine-containing organic residues in an oxygen-rich flame to produce combustion gases which include hydrogen chloride and water vapour and which are fed to an oxychlorination plant for reacting an unsaturated organic compound with oxygen and hydrogen chloride, characterised in that the combustion chamber has means for controlling the temperature of the effluent combusted gases such that they are transferred to the oxychlorination plant at a temperature above the dew point of the water vapour therein.

21. Apparatus according to claim 20 characterised in that the combustion chamber has an outlet for the combusted gases which is connected directly to an inlet port in the oxychlorination plant by lagged ducting.

22. Apparatus according to claim 21, characterised in that the lagged ducting is heat-traced.

23. Apparatus according to any one of claims 20 to 22 characterised in that the means for controlling the temperature of the effluent combusted gases is also capable of controlling that temperature below the limiting temperature of the outlet ducting material when in contact with the effluent gases.

24. Apparatus according to any one of claims 20 to 23 characterised in that it comprises a chamber according to claim 10.

25. Apparatus according to claim 24, characterised in that it comprises a combustion chamber according to claim 11.

26. Apparatus according to claim 24 or 25, characterised in that it comprises a combustion chamber according to claim 12.

27. Apparatus according to any one of claims 24 to 26, characterised in that it comprises a combustion chamber according to claim 13.

28. Apparatus according to any one of claims 20 to 27, characterised in that it comprises a combustion chamber according to claim 14.

29. Apparatus according to claim 28, characterised in that it comprises a combustion chamber according to claim 15.

30. Apparatus according to claim 27, characterised in that it comprises a combustion chamber to claim 16.

31.    Apparatus according to claims 28 to 30 characterised in that it comprises a combustion chamber according to claim 17.

32.    Apparatus according to claim 31, characterised in that it comprises a combustion chamber according to claim 18.

33.    Apparatus according to any one of claims 20 to 32, characterised in that it comprises a combustion chamber according to claim 19.

WATER  STEAM

RESIDUES

O₂

FUEL

3  1  4

2

6

5

8

7  11  9  10  4

14

HCl (B)

HCl (A)

13

12

ETHYLENE
DICHLORIDE

STEAM

O₂

WATER

C₂H₄

1/1

0132971